Europäisches Patentamt

European Patent Office

Office européen des brevets

(11) Veröffentlichungsnummer: **0 221 006**
**B1**

# EUROPÄISCHE PATENTSCHRIFT

(45) Veröffentlichungstag der Patentschrift:
**20.06.90**

(21) Anmeldenummer: **86730169.9**

(22) Anmeldetag: **22.10.86**

(51) Int. Cl.⁵: **F16B 2/12**

(54) **Klammer.**

(30) Priorität: **23.10.85  DE 3538202**

(43) Veröffentlichungstag der Anmeldung:
**06.05.87 Patentblatt 87/19**

(45) Bekanntmachung des Hinweises auf die Patenterteilung:
**20.06.90 Patentblatt 90/25**

(84) Benannte Vertragsstaaten:
**AT BE CH DE ES FR GB GR IT LI LU NL SE**

(56) Entgegenhaltungen:
**AT-B- 362 123
US-A- 643 533
US-A- 791 331
US-A- 1 223 710**

(73) Patentinhaber: **Heidt, Geza, Dr., Richard Strauss-Str. 26,
D-1000 Berlin 33(DE)**

(72) Erfinder: **Koppan, Josef, Kurfürstenstrasse 88,
D-1000 Berlin 30(DE)**

(74) Vertreter: **Lüke, Dierck-Wilm, Dipl.-Ing.,
Gelfertstrasse 56, D-1000 Berlin 33(DE)**

ACTORUM AG

## Beschreibung

Die Erfindung bezieht sich auf eine Klammer gemäß dem Oberbegriff des Patentanspruches 1.

Derartige Klammern werden ganz allgemein verwendet, z.B. als Wäscheklammern, Chirurgieklammern, Lakenklammern, Aktenklammern, als Klammern für fotografische Zwecke, bei Schaufenster-Dekorationen, zur Zusammenfassung von Schriftstücken und dgl.

Die herkömmliche Klammer, auch als Wäscheklammer bezeichnet, besteht aus mit den Betätigungselementen versehenen Klemmbacken, insbesondere aus Holz oder Kunststoff. Als elastisches Element zum Zusammenhalten und Zusammendrücken der bekannten Klammer wird eine metallische Feder verwendet. Durch Zusammendrücken der beiden als Betätigungselemente dienenden Klammergriffe öffnet sich die Klammer und schließt sich nach dem Freilassen der Klammergriffe, wobei der zwischen den Klemmbacken befindliche Gegenstand mittels der Feder zusammengedrückt wird. Nachteilig bei den bekannten Klammern ist der Zusammenbau der beiden, mit den Betätigungselementen versehenen Klemmbacken mittels der besonders gestalteten Feder, welche mit ihren abgewinkelten Federenden in äußere Nuten beider Klemmbacken eingelegt werden muß, wobei sich der Federkörper selbst zwischen den beiden Klemmbacken befindet und die Schwenkachse der beiden Klemmbacken bildet. Ein weiterer Nachteil besteht darin, daß der Druck der beiden Klemmbacken keilförmig wirkt, d.h. eine Tendenz hat, den zu klammernden Gegenstand aus dem Zwischenraum zwischen den Klemmbacken hinauszuschieben. Aus diesem Grunde ist die Klemmwirkung der bekannten Klammer unsicher. Ferner können die beiden Klemmbacken beim Zusammendrücken dickerer Gegenstände von dem eigentlichen Körper der Feder abgehoben werden, so daß diese ohne Führung frei zwischen den beiden Klemmbacken hängt. Ein weiterer Nachteil besteht noch bei der Klammer aus Kunststoff, welche aufgrund einer durch die Anordnung der Feder bedingten Materialschwächung leicht in ihrer Kippachse, d.h. im Bereich zwischen Klemmbacken und Betätigungselement, bricht.

Der Erfindung liegt von daher die Aufgabe zugrunde, eine Klammer der gattungsgemäßen Art dahingehend auszubilden, daß einerseits eine höhere Stabilität und andererseits eine sichere Klemmwirkung der Klemmbacken erzielt wird.

Die Lösung dieser Aufgabe ergibt sich aus den kennzeichnenden Merkmalen des Patentanspruches 1. Die erfindungsgemäße Klammer arbeitet nicht mehr mit einer Schwenkbarkeit der Klemmbacken gegeneinander, sondern mit einer geradlinigen Bewegung der Klemmbacken aufeinander zu, so daß der zusammenzudrückende Gegenstand sicher gehalten wird. Die Wirkungsrichtung verläuft genau senkrecht zur Klemmebene. Ebenfalls senkrecht zur Klemmebene erfolgt eine Öffnungsbewegung der erfindungsgemäßen Klammer, wobei die über das rückwärtige Ende des jeweils anderen Betätigungselementes herausragenden Betätigungsenden jedes Betätigungselementes gegeneinander gedrückt werden und somit durch eine reine Schiebebewegung die Öffnung der Klammer in zur Klemmebene senkrechter Richtung bewirken.

In der bevorzugten Ausführungsform ist ein Betätigungselement mit einer in der Schiebeebene liegenden Führungsnut für einen Führungssteg des anderen Betätigungselementes versehen, welcher in der Führungsnut gleitbar geführt ist. Zum Öffnen und Schließen der Klammer erfolgt eine Relativbewegung von Führungssteg und Führungsnut, welche den parallelen Druck an den Klemmbacken hervorrufen.

Der Zusammenhalt beider Klemmbacken wird in weiterer bevorzugter Ausgestaltung der Erfindung durch einen als elastisches Element wirkenden Gummiring geschaffen, welcher in einem Halsteil zwischen den Klemmbacken und den Betätigungselement innerhalb einer umlaufenden Rille aufgenommen ist.

Die Gleitbewegung der Betätigungselemente der Klemmbacken garantiert einen parallelen, gleichmäßigen Druck der Klemmbacken, welcher auch bei unterschiedlich starken, zusammenzudrückenden Gegenständen in gleicher Kraft und Richtung aufrechterhalten ist.

Ein besonderer Vorteil in der erfindungsgemäßen Ausgestaltung der Klammer liegt darin, daß die Länge der Klemmbacken, je nach Verwendungszweck, beliebig ausgestaltet werden kann, ohne daß Schwierigkeiten mit der Ausbildung der Betätigungselemente bestehen. Bei den im Stand der Technik vorbekannten Klammern muß die Länge der als Betätigungselemente dienenden Griffe immer etwa gleich lang sein wie die Länge der Klemmbacken, jeweils gerechnet vom Drehpunkt der vorbekannten Klammer.

Die mit den Betätigungselementen versehenen Klemmbacken werden in zweckmäßiger Weise aus Kunststoff- Spritzgußmaterial hergestellt. Als besonders vorteilhaft hat sich Polystyrol erwiesen.

Die Erfindung ist nachfolgend anhand eines in den Zeichnungen dargestellten Ausführungsbeispieles näher erläutert. Es zeigen:

Fig. 1 eine Hauptansicht der geschlossenen Klammer,
Fig. 2 eine Seitenansicht hierzu,
Fig. 3 eine Hauptansicht der geöffneten Klammer,
Fig. 4 eine Hauptansicht der linken Klemmbacke mit Betätigungselement mit einer Führungsnut,
Fig. 5 eine Seitenansicht hierzu,
Fig. 6 eine Untersicht,
Fig. 7 eine Hauptansicht der rechten Klemmbacke mit Betätigungselement mit Führungssteg,
Fig. 8 eine Seitenansicht hierzu und
Fig. 9 eine Untersicht hierzu.

Die Klammer besteht aus zwei Klemmbacken 1,2, welche jeweils mit einem Betätigungselement 3 bzw. 4 versehen sind, und einem diese in der Klemmebene 5 zusammendrückenden elastischen Element in Form eines Gummiringes 6. Die Klemmbacken 1,2 sind - mit Ausnahme der Betätigungselemente 3,4 - identisch ausgebildet und umfassen in der Klemmebene 5 gegeneinander drückende Druckflächen 7 und daran anschließende, mit Zähnen 8 versehene Mulden 9, sowie darunter ausgebildete halbkreisförmige Aussparungen 10, welche z.B. bei der Verwendung als Wäscheklammer der Aufnahme der nicht dargestellten Wäscheleine dienen. Auf den jeweiligen Außenseiten sind die Klemmbacken 1,2 mit Aussparungen 11 zur Gewichtsersparnis versehen.

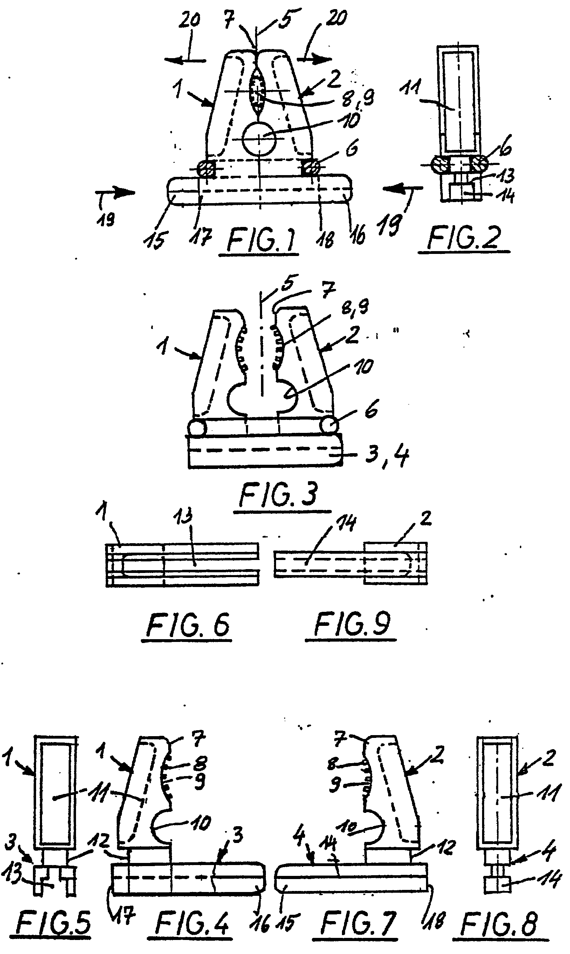

Unterhalb der halbkreisförmigen Aussparungen 10 ist im Bereich eines Halsteiles zwischen den Klemmbacken 1, 2 und den zugehörigen Betätigungselementen 3,4 eine Rille 12 ausgeformt, welche zur Aufnahme des Gummiringes 6 dient, wie es in den Fig. 1 bis 3 näher dargestellt ist.

Die in den Figuren jeweils links dargestellte Klemmbacke 1 umfaßt das Betätigungselement 3, welches sich in einer senkrecht zur Klemmebene 5 gerichteten Ebene erstreckt und ein gabelartiges Schiebeelement ( Fig. 6) mit einer Führungsnut 13 bildet, welche insbesondere aus der Fig. 5 ersichtlich ist. Die Führungsnut 13 ist im Querschnitt T-förmig ausgebildet und erstreckt sich quer durch das Betätigungselement 3, wobei die Führungsnut 13 auch unterhalb der Klemmbacke 1 hindurchgeführt ist.

Die in den Figuren rechts dargestellte Klemmbacke 2 besitzt das in einer senkrecht zur Klemmebene 5 gerichtete Betätigungselement 4, welches als T-förmiger Führungssteg 14 ausgebildet ist, welcher insbesondere in Fig. 8 dargestellt ist und welcher sich auch unterhalb der Klemmbacke 2 erstreckt.

Sowohl die in den Figuren links dargestellte Klemmbacke 1 mit zugehörigem Betätigungselement 3, als auch die in den Figuren rechts dargestellte Klemmbacke 2 mit zugehörigen Betätigungselement 4 sind jeweils einstückig aus Kunststoff, insbesondere Kunststoff-Spritzguß ausgeformt.

Wie es die Fig. 1 zeigt,wird zur Montage der Klammer die rechte Klemmbacke 2 mit ihrem Führungssteg 14 in die Führungsnut 13 der linken Klemmbacke 1 eingeschoben, wobei das Betätigungsende 15 des Führungssteges 14 der rechten Klemmbacke 2 aus dem rückwärtigen Ende 17 der linken Klemmbacke 1 herausragt, wie es in Fig. 1 dargestellt ist. In entsprechender Weise ragt das Betätigungsende 16 des Betätigungselementes 3 nach rechts über das rückwärtige Ende 18 der rechten Klemmbacke 2 hinaus, wie es ebenfalls in Fig. 1 dargestellt ist. Anschließend wird der Gummiring 6 über die Klemmbacken 1,2 hinweg in die Rille 12 eingebracht. Durch Fingerdruck, z.B. vom Daumen und Zeigefinger einer Hand, in Richtung der Pfeile 19 ( Fig.1) kann die Klammer geöffnet werden, wobei die Klemmbacken 1,2 jeweils in Richtung der Pfeile 20 auseinander bewegt werden. Die maximale Öffnungsweite ist dann erreicht, wenn die äußere Kontur der Betätigungsenden 15,16 in Deckung mit den rückwärtigen Enden 17 bzw. 18 der jeweiligen Betätigungselemente 3 bzw. 4 liegen.

In einer nicht näher dargestellten Ausführungsform können die Betätigungselemente 3,4 auch mit Griffnasen versehen sein, welche in Richtung der Klemmebene 5 unter den Betätigungselementen 3, 4 herausragen, so daß eine größere Öffnungsweite erzielt werden kann.

Die Länge der Klemmbacken 1,2 ist wahlfrei und kann an die benötigte Länge für den jeweiligen Einsatzzweck angepaßt werden.

## Patentansprüche

1. Klammer aus zwei mit Betätigungselementen versehenen, insbesondere aus Kunststoff ausgeformten Klemmbacken und aus einem diese verbindenden und in der Klemmebene zusammendrückenden elastischen Element, **dadurch gekennzeichnet,** daß die Betätigungselemente (3,4) in einer senkrecht zur Klemmebene (5) gerichteten Ebene gegen die Wirkung des elastischen Elementes (6) schiebbar angeordnet sind und daß zumindest im geschlossenen Zustand der Klammer das Betätigungsende (15, 16) eines jeden Betätigungselementes (3,4) über das rückwärtige Ende (17 bzw. 18) des jeweils anderen Betätigungselementes (1,2) hinausragt.

2. Klammer nach Anspruch 1, dadurch gekennzeichnet, daß ein Betätigungselement (3) in der Schiebeebene mit einer Führungsnut (13) und das andere Betätigungselement (4) mit einem in dieser gleitbar geführten Führungssteg (14) versehen ist.

3. Klammer nach Anspruch 2, dadurch gekennzeichnet, daß der Querschnitt von Führungsnut (13) und Führungssteg (14) T- förmig ausgebildet ist.

4. Klammer nach einem der Ansprüche 1 bis 3, dadurch gekennzeichnet, daß als elastisches Element ein in einem Halsteil zwischen den Klemmbacken (1, 2) und den Betätigungselementen (3,4) in einer umlaufenden Rille (12) aufgenommener Gummiring (6) vorgesehen ist.

## Claims

1. A clamp comprising two clamping jaws formed in particular from plastic and provided with actuating members, and comprising an elastic member joining the latter and pressing them together in the clamping plane, characterised by that the actuating members (3, 4) are arranged slidably against the action of the elastic member (6) in a plane directed vertically to the clamping plane (5), and that at least in closed condition of the clamp, the actuating end (15, 16) of each actuating member (3, 4) projects over the rear-side end (17 or 18, resp.) of the respective other actuating member (1, 2).

2. A clamp according to claim 1, characterised by that one actuating member (3) is provided in the sliding plane with a guide groove (13), and that the other actuating element (4) is provided with a guide projection (14) to be guided within said guide groove.

3. A clamp according to claim 2, characterised by that the cross-sections of guide groove (13) and guide projection (14) are T-shaped.

4. A clamp according to one of claims 1 to 3, characterised by that as elastic member, a rubber ring (6) accommodated in a neck portion between the clamping jaws (1, 2) and the actuating members (3, 4) in a circumferential groove (12) is provided.

**Revendications**

1. Bride comportant deux coussinets de serrage pourvus d'éléments d'actionnement et formés particulièrement de plastique et comportant un élément élastique joignant ceux-ci et les comprimant dans le plan de serrage, caractérisée en ce que les éléments d'actionnement (3, 4) sont agencés en manière coulissante dans un plan perpendiculaire sur le plan de serrage (5) contre l'action de l'élément élastique (6), et qu'au moins en état fermé de la bride l'extrémité d'actionnement (15, 16) de chaque élément d'actionnement (3, 4) dépasse l'extrémité arrière (17 ou 18) du respectivement autre élément d'actionnement (1, 2).

2. Bride selon la revendication 1, caractérisée en ce qu'un élément d'actionnement (3) est pourvu dans le plan de glissage d'une rainure de guidage (13) et que l'autre élément d'actionnement (4) est pourvu d'une saillie de guidage (14) guidée en manière glissante là-dedans.

3. Bride selon la revendication 2, caractérisée en ce que les sections transversales de la rainure de guidage (13) et de la saillie de guidage (14) sont agencées en forme de T.

4. Bride selon une des revendications 1 à 3, caractérisée en ce que comme élément élastique, une rondelle de caoutchouc (6) agencée dans une partie à gorge entre les coussinets de serrage (1, 2) et les éléments d'actionnement (3, 4) dans une rainure circonférentielle (12) est prévue.

FIG.1

FIG.2

FIG.3

FIG.6

FIG.9

FIG.5

FIG.4

FIG.7

FIG.8